# EUROPEAN PATENT APPLICATION

(11) **EP 3 211 067 A1**
(43) Date of publication of application: **30.08.2017**
(21) Application number: 17000274.5
(22) Date of filing: 21.02.2017
(51) Int. Cl.: C12N 1/20, C12Q 1/00, G01N 1/28

(54) **SELECTIVE MEDIUM FOR CULTIVATING PATHOGENIC FUNGI**

(30) Priority: 25.02.2016 EP 16000462
(71) Applicant: Tietz, Ninel, 10117 Berlin (BE)
(72) Inventor: Tietz, Hans-Jürgen, 10117 Berlin (DE)
(74) Representative: Müller-Boré & Partner Patentanwälte PartG mbB

(57) **Abstract**

The present invention relates to a medium for cultivating at least one pathogenic fungus, said medium containing polymyxin E, a method for cultivating at least one pathogenic fungus containing the step of growing the pathogenic fungus on said medium as well as a use of said medium for cultivating at least one pathogenic fungus.

## Description

The present invention relates to a medium for cultivating at least one pathogenic fungus, said medium containing polymyxin E, a method for cultivating at least one pathogenic fungus containing the step of growing the pathogenic fungus on said medium, as well as a use of said medium for cultivating at least one pathogenic fungus.

In order to identify pathogenic fungi, an isolated growth of the fungus on a medium is necessary. Patient samples, however, very often contain numerous bacteria and non-pathogenic fungi, like for example mold fungus, which overgrow the pathogenic fungus of interest during cultivation. One of the problems arising from using antibiotics in media for growing pathogenic fungi is the fact that many antibiotics also suppress the growth of pathogenic fungi.

Thus, the problem underlying the present invention is to provide a new medium for cultivating pathogenic fungi which enables a growth of the pathogenic fungus of interest without it being overgrown by bacteria or non-pathogenic fungi.

The solution to the above technical problem is achieved by the embodiments characterized in the claims.

In particular, the present invention relates to a medium for cultivating at least one pathogenic fungus, said medium containing polymyxin E. In a preferred embodiment of the present invention, the medium contains 1 to 200 mg/liter, more preferably 1 to 100 mg/liter, more preferably 1 to 80 mg/liter, more preferably 1 to 50 mg/liter, more preferably 1 to 40 mg/liter, more preferably 2 to 20 mg/liter, more preferably 5 to 15 mg/liter, more preferably 8 to 12 mg/liter, and most preferably about 10 mg/liter polymyxin E.

In a preferred embodiment of the present invention, the medium further contains chloramphenicol. Preferably, the medium contains 1 to 300 mg/liter, more preferably 1 to 200 mg/liter, more preferably 1 to 100 mg/liter, more preferably 10 to 100 mg/liter, more preferably 10 to 80 mg/liter, more preferably 20 to 80 mg/liter, more preferably 40 to 60 mg/liter, more preferably 45 to 55 mg/liter, and most preferably about 50 mg/liter chloramphenicol.

In another preferred embodiment of the present invention, the medium further contains cycloheximide. Preferably, the medium contains 50 to 2000 mg/liter, more preferably 50 to 1000 mg/liter, more preferably 50 to 800 mg/liter, more preferably 100 to 800 mg/liter, more preferably 200 to 800 mg/liter, more preferably 200 to 600 mg/liter, more preferably 300 to 500 mg/liter, more preferably 350 to 450 mg/liter, and most preferably about 400 mg/liter cycloheximide.

In another preferred embodiment of the present invention, the medium further contains peptone, glucose and/or agar-agar which may be derived from any available source. Preferably, the medium contains 1 to 100 g/liter, more preferably 1 to 80 g/liter, more preferably 1 to 50 g/liter, more preferably 5 to 30 g/liter, more preferably 5 to 20 g/liter, and most preferably about 10 g/liter peptone. Preferably, the medium contains 2 to 200 g/liter, more preferably 2 to 100 g/liter, 2 to 80 g/liter more preferably 5 to 60 g/liter, more preferably 10 to 40 g/liter, more preferably 10 to 30 g/liter and most preferably about 20 g/liter glucose. Preferably, the medium contains 2 to 200 g/liter, more preferably 2 to 100 g/liter, 2 to 80 g/liter more preferably 5 to 60 g/liter, more preferably 10 to 40 g/liter, more preferably 10 to 30 g/liter and most preferably about 20 g/liter agar-agar.

In a particularly preferred embodiment of the present invention, the medium contains 10 g/liter peptone, 20 g/liter glucose, 400 mg/liter cycloheximide, 10mg/liter polymyxin E, and 20 g/liter agar-agar.

The medium may be any medium that is known from the literature or commercially available that is suitable for cultivating at least one pathogenic fungus and further contains polymyxin E. The medium can additionally comprise a number of ingredients, including amino acids, vitamins, organic and inorganic salts, sources of carbohydrate, each ingredient being present in an amount which supports the cultivation of at least one pathogenic fungus. The medium may contain auxiliary substances, such as buffer substances like sodium bicarbonate.

In a preferred embodiment of the present invention the medium is solid. The solid medium can be present on any type of solid carrier. The term "solid carrier" does not have any specific limitations, and relates, for example, to glass or an insoluble polymer material, which can be an organic polymer, such as polyamide or a vinyl polymer (e.g. poly(meth)acrylate, polystyrene and polyvinyl alcohol, or derivatives thereof), a natural polymer such as cellulose, dextran, agarose, chitin and polyamino acids, or an inorganic polymer, such as glass or metallohydroxide. In a preferred embodiment of the present invention, the solid carrier is a petri dish.

In a preferred embodiment of the present invention, the pathogenic fungus is derived from a sample taken from a patient. The term "patient" as used herein comprises any patient. In a preferred embodiment of the present invention, the patient is a vertebrate, more preferably a vertebrate selected from the group consisting of fish, birds, reptiles, and mammals. In a preferred embodiment of the present invention, the patient is a mammal, preferably a mammal selected from the group consisting of human, mouse, rat, pig, cat, dog, horse, goat, cattle, cow, and monkey and/or others. In a most preferred embodiment of the present invention, the patient is a human.

The term "sample" as used herein, comprises any sample. In one embodiment of the present invention, the sample may be derived from a naturally occurring system, preferably a sample containing a body fluid or components derived from a body fluid, a sample containing a body tissue or components derived from a body tissue, or a sample containing a smear or components derived from a smear. A body fluid or a component derived therefrom may be of various origins, preferably from serum, saliva, urine, bile, lymph, tissue, like e.g. bladder or kidney, cerebrospinal fluid and/or other body fluids in the case of an animal or human individual. A body tissue or a component derived therefrom may be of various origins, preferably from muscle tissue, heart tissue, liver tissue, kidney tissue, epithelial tissue, skin, connective tissue, or nervous tissue. A smear or a component derived therefrom may be of various origins, preferably from inorganic surfaces, wounds, epithelium including for example epithelium in the mouth, the urethra, the skin, glands, the after, the eye, or the vagina.

In a preferred embodiment of the present invention, the sample is a smear taken from skin and the pathogenic fungus is a dermatophyte.

The term "pathogenic fungus" as used herein comprises any fungus that causes a mycosis including superficial mycoses, cutaneous mycoses, subcutaneous mycoses, and systemic mycoses. In a preferred embodiment, the pathogenic fungus is a dermatophyte. In another preferred embodiment, the pathogenic fungus is selected from the group consisting of *Epidermophyton ssp., Microsporum ssp., Trichophyton ssp., Coccidioides ssp., Histoplasma ssp., Candida ssp., Aspergillus ssp.*, preferably excluding *Aspergillus niger*, and *Cryptococcus ssp.* In a more preferred embodiment, the pathogenic fungus is selected from the group consisting of *Candida albicans* (e.g. *C. albicans* strain ATCC 10231), *Trichophyton mentagrophytes* (e.g. *T. mentagrophytes* strain ATCC 9533), *Trichophyton tonsurans, Microsporum canis* (e.g. *M. canis* strain ATCC 36299), *Microsporum gypseum*, and *Scopulariopsis brevicaulis*.

In a preferred embodiment of the present invention, the medium according to the present invention prevents the growth of the bacteria *Escherichia coli* (e.g. *E. coli* strain ATCC 25922) and *Pseudomonas aeruginosa* (e.g. *P. aeruginosa* strains ATCC 10145, ATCC 27853, or ATCC 9027), as well as the growth of the non-pathogenic fungus *Aspergillus niger* (e.g. *A. niger* strain ATCC 16404).

The present invention further relates to a method for cultivating at least one pathogenic fungus as defined herein comprising the step of growing the pathogenic fungus on the medium as defined herein. In a preferred embodiment of the present invention, the pathogenic fungus is derived from a sample as defined herein taken from a patient as defined herein.

According to the present invention, concurrently with cultivating the at least one pathogenic fungus, the growth of bacteria and non-pathogenic fungi is suppressed. In this context, the bacteria and non-pathogenic fungi are preferably as defined above. In a preferred embodiment of the present invention, the method according to the present invention comprises the step of processing the sample before cultivating the pathogenic fungus on the medium.

In another preferred embodiment of the present invention, the step of cultivating at least one pathogenic fungus is preferably carried out at 18°C to 42°C, more preferably at 21 °C to 40°C, more preferably at 26°C to 40°C, more preferably at 30°C to 40°C, more preferably at 35°C to 39°C, more preferably at 36°C to 38°C, and most preferably at 37°C, preferably for 6 to 240 hours, more preferably for 6 to 200 hours, more preferably for 6 to 180 hours, more preferably for 6 to 120 hours, more preferably for 6 to 80 hours, more preferably for 8 to 60 hours, more preferably for 12 to 60 hours, more preferably for 24 to 48 hours, more preferably for 30 to 40 hours.

The present invention further relates to a use of a medium as defined herein for cultivating at least one pathogenic fungus as defined herein.

Any limitations, definitions and preferred embodiments defined above apply to this aspect of the present invention in an analogous manner.

According to the present invention, a medium is provided that advantageously allows for the cultivation of clinically important pathogenic fungi such as dermatophytes, in particular those dermatophytes indicated above, while at the same time suppressing the growth of bacteria and non-pathogenic fungi that would otherwise overgrow the pathogenic fungi of interest. The present invention has identified a medium that advantageously and surprisingly allows for this distinction between pathogenic fungi of interest and bacteria and non-pathogenic fungi that would impede cultivation of the pathogenic fungi of interest as far as growth on the medium is concerned.

The figures show:
Figure 1: Growth of *Trichophyton tonsurans* on a medium containing 10 g/liter peptone, 20 g/liter glucose, 400 mg/liter cycloheximide, 10mg/liter polymyxin E, and 20 g/liter agar-agar in 1000 ml aqua dest.
Figure 2: Growth of *Trichophyton mentagrophytes* strain ATCC 9533 on media according to the present invention, comprising 12,5 mg/liter, 14 mg/liter and 16 mg/liter polymyxin E, respectively, as compared to growth on a conventional dermatophyte agar.
Figure 3: Growth of *Microsporum canis* strain ATCC 36299 on media according to the present invention, comprising 12,5 mg/liter, 14 mg/liter and 16 mg/liter polymyxin E, respectively, as compared to growth on a conventional dermatophyte agar.
Figure 4: Master cultures of *Pseudomonas aeruginosa, Microsporum canis, Microsporum gypseum, Trichophyton mentagrophytes,* and *Scopulariopsis brevicaulis* (from left to right).
Figure 5: Combinations of *Pseudomonas aeruginosa* and pathogenic fungi to be tested in suspension prior to inoculation of the test media.
Figure 6: *Pseudomonas aeruginosa* almost completely suppresses the growth of *Microsporum canis* without addition of polymyxin E, so that only one colony of *M. canis* is obtained.
Figure 7: Polymyxin E completely suppresses the growth of *Pseudomonas aeruginosa.* In contrast, *Microsporum canis* is growing in polymyxin E concentrations of 10 mg/liter and 12,5 mg/liter without losing any of its species-specific micro- and macroscopic characteristics, such as the typical golden-yellow pigment.

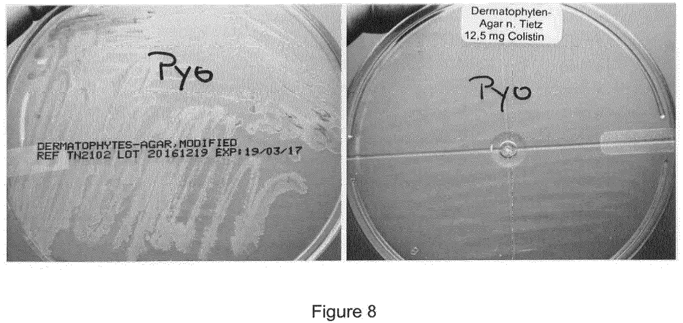

Figure 8: Growth of *Pseudomonas aeruginosa* on conventional dermatophyte agar (left) and complete suppression thereof on a medium of the present invention, comprising 12,5 mg/liter polymyxin E (right).

The present invention will now be further illustrated in the following example without being limited thereto.

### Examples

### Example 1:

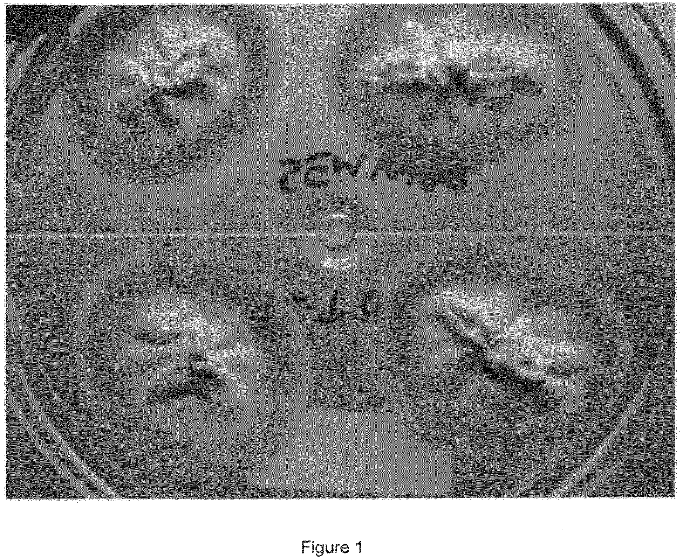

A sample provided by a patient is given on a petri dish containing the following medium: 10 g/liter peptone, 20 g/liter glucose, 400 mg/liter cycloheximide, 10mg/liter polymyxin E, 20 g/liter agar-agar in 1000 ml aqua dest. The medium is divided in four sections and a fragment of the sample is given in each section. After an incubation at 37°C in an incubator for a few days a growth of *Trichophyton tonsurans* can be seen (cf. Fig. 1).

The displayed shown in Fig. 1 clearly shows that *Trichophyton tonsurans* can be identified without any contamination by bacteria or mold fungus. The composition of the medium does not inhibit the growth of *Trichophyton tonsurans.*

### Example 2:

A conventional modified dermatophyte agar (TN1054) and media according to the present invention, based on said conventional agar and comprising various amounts of polymyxin E, were generated. Briefly, the ingredients were solubilized by cooking, the resulting media autoclaved for 15 min at 121 °C, and about 20 ml of the media poured into petri dishes.

The media were clear and of a slightly yellowish color. The pH value was found to be in a range of pH 5.5 to pH 5.9. The media displayed a gel strength of at least 3.8 N/cm² or more.

For assessing the ability of various pathogenic and non-pathogenic fungi, as well as various bacteria, to grow on the media of the present invention, two petri dishes per organism to be tested were directly inoculated from a master culture and the dishes incubated under aerobic conditions for 2 to 7 days at 25 to 30 °C. Growth and colony morphology is given in the Table 1 below.

**Table 1**

| **Test strain** | **Growth / colony morphology** |
|---|---|
| *Candida albicans* | Solid growth |
| ATCC 10231 | White, dry colonies |
| *Trichophyton mentagrophytes* | Solid growth |
| ATCC 9533 | White mycelium with powdery surface |
| *Microsporum canis* | Solid growth |
| ATCC 36299 | White, partially fringed mycelium |
| *Aspergillus niger* | No growth |
| ATCC 16404 | |
| *Escherichia coli* | No growth |
| ATCC 25922 | |
| *Pseudomonas aeruginosa* | No growth |
| ATCC 10145 | |
| *Pseudomonas aeruginosa* | No growth |
| ATCC 27853 | |
| *Pseudomonas aeruginosa* | No growth |
| ATCC 9027 | |

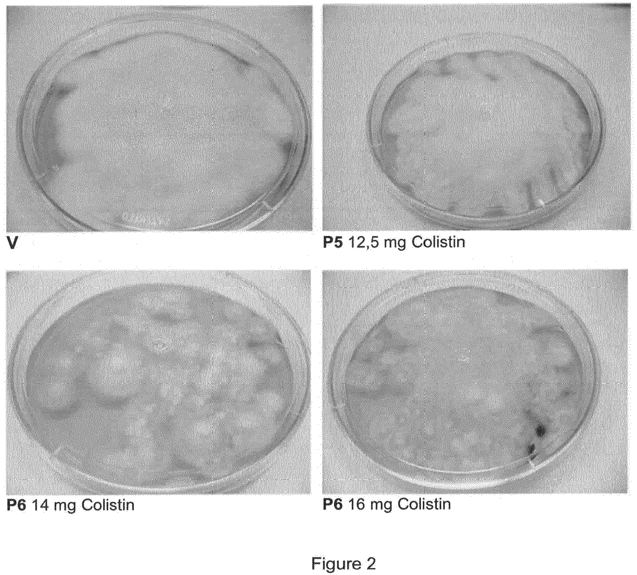

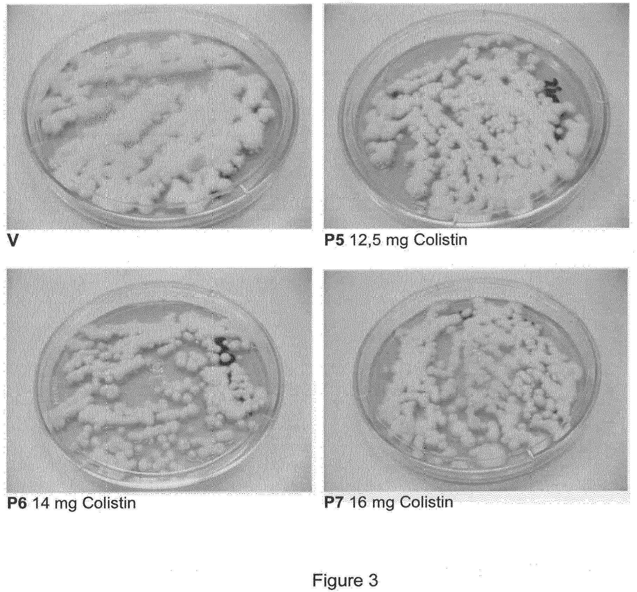

Mycelium growth of test strain *Trichophyton mentagrophytes* ATCC 9533 decreases with increasing polymyxin E concentrations, starting at concentrations of 12,5 mg/liter polymyxin E (Fig. 2). Test strain *Microsporum canis* ATCC 36299 displays no optically significant changes with increasing polymyxin E concentrations (Fig. 3).

Further, both test strains *Pseudomonas aeruginosa* ATCC 27853 and *Pseudomonas aeruginosa* ATCC 9027 show no growth at a polymyxin E concentration of 10 mg/liter. Further, growth of test strain *Pseudomonas aeruginosa* ATCC 10145 is completely suppressed at a polymyxin E concentration of 16 mg/liter.

Furthermore, test strains *Aspergillus niger* ATCC 16404 and *Escherichia coli* ATCC 25922 do not show any growth.

### Example 3:

*Pseudomonas aeruginosa* is a mostly non-pathogenic germ common in moist areas that often colonizes human skin and nails. It poses a big problem in mycological diagnosis, since it inhibits and/or completely overgrows the culture of dermatophytes on all known media. Many media contain additives such as chloramphenicol, in order to control contaminants. However, *P. aeruginosa* is often chloramphenicol resistant and can grow on such media.

In order to overcome this situation, the present invention provides media comprising polymyxin E, which advantageously inhibit the growth of Pseudomonads but do not impair the culture of dermatophytes.

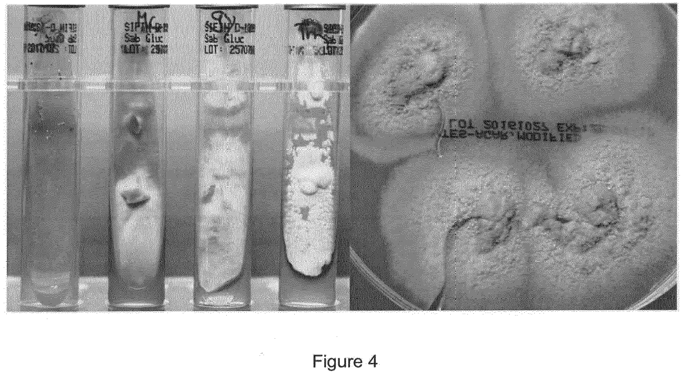

In order to evaluate respective media, two batches of a media based on a conventional modified dermatophyte agar (TN1054) and further containing 10 mg/liter and 12,5 mg/liter polymyxin E, respectively, where prepared. Test organisms were *Pseudomonas aeruginosa, Microsporum canis, Microsporum gypseum, Trichophyton mentagrophytes,* and *Scopulariopsis brevicaulis* (Fig. 4).

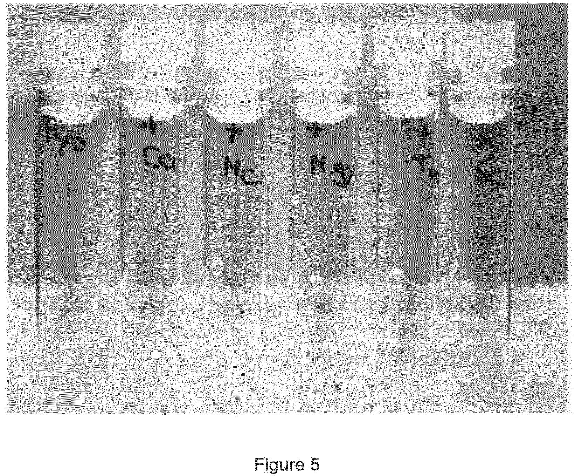

The test organisms were inonculated onto said media, as well as onto a control medium not containing polymyxin A, as pure cultures or as combinations of test organisms in suspension (Fig. 5). The growth of the test organisms was evaluated micro- and macroscopically after 5 and 10 days and documented photographically.

Results of these experiments are shown in Table 2 below.

**Table 2:**

| **Test organism** | **Medium** | | |
|---|---|---|---|
| | **TN 1054** | **TN 1054 + 10 mg/l polymyxin E** | **TN 1054 + 12,5 mg/l polymyxin E** |
| *P. aeruginosa* | + | - | - |
| *C. albicans* | + | + | + |
| *M. canis* | + | + | + |
| *M. gypseum* | + | + | + |
| *T. mentagrophytes* | + | + | + |
| *S. brevicaulis* | + | + | + |
| *P. aeruginosa & C. albicans* | + | - | - |
| | + | + | + |
| *P. aeruginosa & M. canis* | + | - | - |
| | suppressed | + | + |
| *P. aeruginosa & M. gypseum* | + | - | - |
| | suppressed | + | + |
| *P. aeruginosa & T. menatgrophytes* | + | - | - |
| | suppressed | + | + |
| *P. aeruginosa & S. brevicaulis* | + | - | - |
| | suppressed | + | + |

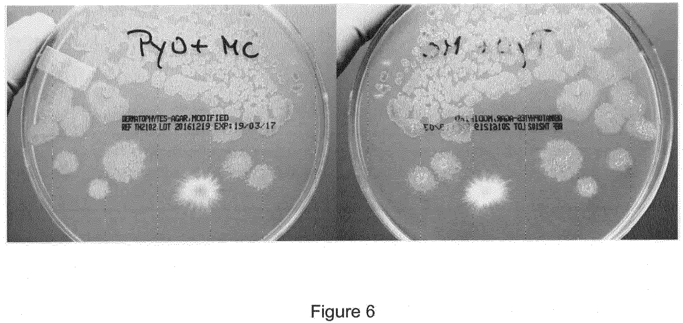

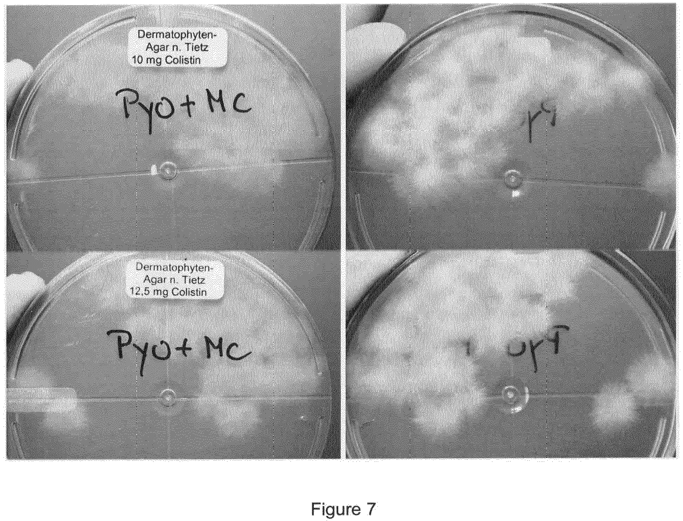

As a result, *Pseudomonas aeruginosa* suppresses the growth of dermatophytes without addition of polymyxin E (Fig. 6). Further, this suppression is abrogated by polymyxin E (Fig. 7). Furthermore, media comprising polymyxin E completely suppress the growth of *P. aeruginosa*.

## Claims

1. A medium for cultivating at least one pathogenic fungus, said medium containing polymyxin E.

2. The medium according to claim 1, further containing chloramphenicol.

3. The medium according to claim 1 or 2, further containing cycloheximide.

4. The medium according to any of claims 1 to 3, further containing peptone, glucose and/or agar-agar.

5. The medium according to any of claims 1 to 4, containing 1 to 50 mg/liter polymyxin E.

6. The medium according to any of claims 1 to 5, containing 2 to 20 mg/liter polymyxin E.

7. The medium according to any of claims 1 to 6, containing 10 to 100 mg/liter chloramphenicol.

8. The medium according to any of claims 1 to 7, containing 40 to 60 mg/liter chloramphenicol.

9. The medium according to any of claims 1 to 8, containing 50 to 1000 mg/liter cycloheximide.

10. The medium according to any of claims 1 to 9, containing 200 to 600 mg/liter cycloheximide.

11. A method for cultivating at least one pathogenic fungus comprising the step of growing the pathogenic fungus on the medium according to any of claims 1 to 10.

12. The method according to claim 11, wherein concurrently with cultivating the at least one pathogenic fungus, the growth of bacteria and non-pathogenic fungi is suppressed.

13. The method according to claim 11 or claim 12, wherein the pathogenic fungus is selected from the group consisting of *Candida albicans, Trichophyton mentagrophytes, Trichophyton tonsurans, Microsporum canis, Microsporum gypseum*, and *Scopulariopsis brevicaulis*.

14. The method according to claim 12 or claim 13, wherein the bacteria are selected from the group consisting of *Escherichia coli* and *Pseudomonas aeruginosa,* and the non-pathogenic fungus is *Aspergillus niger*.

15. Use of a medium according to any of claims 1 to 10 for cultivating at least one pathogenic fungus.
